Europäisches Patentamt

European Patent Office

Office européen des brevets

(11) Publication number: **0 519 113 A1**

## EUROPEAN PATENT APPLICATION

(21) Application number: **91110291.1**

(22) Date of filing: **21.06.91**

(51) Int. Cl.⁵: **C12N 1/20**, C12N 15/00, C12P 13/06, C12P 13/08, //(C12N1/20,C12R1:19)

(43) Date of publication of application:
**23.12.92 Bulletin 92/52**

(84) Designated Contracting States:
**BE DE FR GB IT**

(71) Applicant: **AJINOMOTO CO., INC.**
**5-8, Kyobashi 1-chome, Chuo-ku**
**Tokyo 104(JP)**

(72) Inventor: **Livishits, Vitaly Arkadievich**
**c/o VNIIGENETIKA 1Dorozhny proezd,**
**d.1.Moscow, 113545(SU)**
Inventor: **Debabov, Vladimir Georgievich**
**c/o VNIIGENETIKA 1 Dorozhny proezd,**
**d.1. Moscow, 113545(SU)**
Inventor: **Gavrilova, O.F.**
**c/o VNIIGENETIKA 1 Dorozhny proezd,**
**d.1. Moscow, 113545(SU)**
Inventor: **Zakataeva, N.P.**
**c/o VNIIGENETIKA 1 Dorozhny proezd,**
**d.1. Moscow 113545(SU)**
Inventor: **Shakulov, Rustem Saidovich**
**c/o VNIIGENETIKA 1 Dorozhny proezd,**
**d.1. Moscow 113545(SU)**
Inventor: **Bachina, Tatyana Alexandrovna**
**c/o VNIIGENETIKA 1 Dorozhny proezd,**
**d.1. Moscow 113545(SU)**
Inventor: **Khurges, Evgeny Moiseevich**
**c/oVNIIGENETIKA 1 Dorozhny proezd,**
**d.1. Moscow 113545(SU)**

(74) Representative: **Strehl, Schübel-Hopf,**
**Groening**
**Maximilianstrasse 54 Postfach 22 14 55**
**W-8000 München 22(DE)**

(54) **Bacterial strains having high productivity of an amino acid and method of constructing these strains.**

(57) Bacterial strains which produce increased amounts of amino acids comprising in their genome a mutation affecting the corresponding aminoacyl-tRNA synthetase conferring auxotrophy which cannot be fully suppressed by addition of this amino acid into the culture medium in combination with mutations which destroy the negative regulation in the biosynthesis of the selected amino acid.

EP 0 519 113 A1

The present invention relates to the microbiological industry and, more specifically, it relates to a method for preparing strains which produce amino acids.

Amino acids produced by microorganisms find extensive use as feedstuff and food additives in the agriculture and food industry, as components of various nutrient mixtures for medical purposes and as reagents in the chemical and pharmaceutical industries.

Known in the art are methods for preparing strains which produce amino acids such as L-lysine, L-threonine, L-isoleucine, L-valine and the like by using various mutagens. The resulting mutant strains of microorganisms have specific genetically preconditioned defects in regulating metabolism and, due to such defects, they produce specific amino acids or release them into the nutrient medium. The required microorganism strains are produced by conventional methods based on the particular nutritive demand of a mutant (auxotrophy) or on resistance of a mutant with respect to one or another structural analogues of an amino acid inhibiting the growth of the parent strain.

The amino acid production by known auxotrophic strains results from blocking the formation of a by-product or a coinhibitor amino acid, which participate in the negative control of the amino acid biosynthesis.

Known are pantothenate auxotrophs of Escherichia coli, producing valine (Maas W.K., Vogel H.J. J. Bacteriol., V.65. p.388, 1953), homoserine-requiring strains of Corynebacterium glutamicum and Brevibacterium flavum, producing lysine (Nakayama et al., J. Gen. Appl. Microbiol., v.7, p.41, 1961), isoleucine-, threonine-, or homoserine-requiring mutants of Arthrobacter paraffineus and Corynebacterium hydrocarboclastus producing valine (U.S. Patent no. 3,700,556).

The amino acid production by mutant strains resistant to structural analogues of amino acids results from removing the negative regulation of the amino acid biosynthesis (feed-back inhibition of the key enzyme activity or repression of the corresponding enzyme formation by the end products). Known are the S-2-aminoethyl-L-cysteine resistant mutant of genus Brevibacterium producing lysine (Sano K., Shiio I., J. Gen. Appl. Microbiol., v. 16, p. 373, 1970, Shiio, et al., J. Biochem., v. 68, p. 701, 1970), the amino-hydroxyvaleric acid (AHV) resistant mutant of Escherichia coli producing threonine (Shiio I., Nakamoris., Agr. Biol. Chem., v.33, p.1152, 1969), AHV resistant mutant of the Brevibacterium producing threonine and the mutant of microorganisms belonging to the genus Brevibacterium or Corynebacterium resistant to aminohydroxyvaleric acid producing isoleucine (U.S. Patent No. 3,767,529), mutant strains of the genera Brevibacterium and Corynebacterium having resistance to 2-thiazolalanine which produce valine (U.S. Patent No. 3,893,888) mutant strains of Serratia marcescens resistant to isoleucine hydroxamate producing isoleucine (Kisumi, et al., J. Bacteriol., v. 110, p. 761, 1972).

Known are amino acid-producing strains having resistance to amino acid analogues together with a nutrient requirement, which increases their productivity (U.S.Patent No. 3,893,888). Such amino acid producers remain auxotrophic and can grow only on media containing specific additives.

Known in the art is also a method for preparing bacterial strains producing amino acids which is based on isolation of chromosome DNA fragments of a donor bacterium containing genes controlling the synthesis of a selected amino acid, combining them with a multicopy plasmid DNA molecule by in vitro manipulation and transforming with the hybrid DNA molecule obtained a recipient strain to yield a bacterial strain possessing increased productivity (U.S. Patents Nos. 4,278,765; 4,391,907). According to this method chromosomal DNA fragments are isolated from a strain having a mutation which removes the negative regulation of selected amino acid biosynthesis. The recipient strains may be a specially constructed strain or it may be the donor strain.

However, no effective Escherichia coli strains producing isoleucine or valine were obtained by this method.

A need therefore continues to exist for the development of a novel method for preparing amino acid producing strains.

Hitherto unknown are strains characterized by an increased production of an amino acid due to mutation in genes coding for the corresponding aminoacyl-tRNA synthetase.

The aminoacyl-tRNA synthetases, or activating enzymes, are particularly crucial elements in the route leading from amino acids to proteins. These enzymes catalyze the formation of activated amino acids, that is their attachment to one or more specific tRNA.

In most cases there is but one aminoacyl-tRNA synthetase for each amino acid. So, only conditionally expressed mutations may be obtained.

Mutants with altered aminoacyl-tRNA synthetases were described in several papers :

1. Roth j.R., and Ames B.N. J.Mol.Biol., v. 22, p. 325, 1966.

2. Neidhardt F.C. Bacteriol. Rev., 30, p. 701, 1966.

3. Folk W.R. and Berg P.J. Bacteriol., v. 102, p. 193, 1970.

4. Iaccarino M., and Berg P. J. Bacteriol., v. 105, p. 527, 1971.

5. Johnson E.M. et al. J. Bacteriol., v. 129, p. 66, 1979.

Some aminoacyl-tRNA synthetase mutations manifest themselves as auxotrophy. This is an unusual auxotrophy, because the defect is not in the formation of the amino acid, but in its utilisation for protein synthesis. It was also communicated, that phenotypic suppression of such auxotrophic mutations may arise as a result of additional mutations which increase the biosynthesis and intracellular concentration of the corresponding amino acid (1,4-5). However, amino acid producing strains constructed on the basis of auxotrophic aminoacyl-tRNA synthetase mutations and methods for their production were not known. The role of such mutations in overproduction of amino acids by producer strains was not shown as well.

It is an object of the present invention to use aminoacyl-tRNA synthetase mutations for preparing strains possessing enhanced capability of producing amino acids without additional growth factors, more specifically, strains of Escherichia coli producing isoleucine and valine.

These and other objects of the invention, which will hereafter become more readily apparent, have been attained by development of a method for preparing strains which produce amino acids, wherein according to the present invention, a mutation affecting aminoacyl-tRNA synthetase which confers to cells auxotrophy and which can be compensated only partially by addition of the selected amino acid into the culture medium is combined with mutations which remove the negative regulation of the amino acid biosynthesis to give a strain having increased productivity of the selected amino acid.

This invention further relates to bacterial strains having high productivity for an amino acid, comprising in its genome a mutation which affects the corresponding aminoacyl-tRNA synthetase conferring auxotrophy which can be suppressed partially by adding the respective amino acid to the culture medium, in combination with one or more mutations which remove the negative regulation in the biosynthesis of said amino acid.

Mutations affecting aminoacyl-tRNA synthetases which manifest themselves as auxotrophy have often decreased affinity {increased the Km [Michaelis constant (affinity of substrate)]} for the corresponding amino acids (3-4). Therefore the formation of the activated amino acids (the charging of the corresponding tRNA) in their cells is decreased. Under this condition the transcription of the structural genes (operons) involved in the biosynthesis of these amino acids may be markedly increased, if their expression is controlled by attenuation. This regulatory mechanism exists in many amino acid operons of different bacterial species (Kolter R., Yanofsky C. Ann. Rev. Genet., v. 16, p. 113, 1982; Matsui K. et al. Nucleic Acids Res., v. 14, p. 10113, 1986; Shimotsu H. et al. J. Bacteriol., v. 166, p. 461, 1986; Kuroda M.I. et al. J. Bacteriol., v. 167, p. 792, 1986).

Besides, the decreased tRNA charging elevates the intracellular concentration of guanosinetetraphosphate (ppGpp), which is known to activate initiation of transcription of amino acid operons (Stephens, J.C. et al. Proc. Nat. Acad. Sci. USA, v. 72, p. 389, 1975; Cashel M. and Rudd K.E. in : Escherichia coli and Salmonella typhimurium; cellular and molecular biology (Vol. 2. Neidhardt F.C., ed.) p. 1410. ASM, Washington, 1987).

Taken together these factors create the possibility for overproduction of the corresponding amino acid. This possibility can be realized if the negative regulation of the amino acid biosynthesis is eliminated.

To obtain the high level of expression of amino acid operons which is necessary for effective amino acid production, only those aminoacyl-tRNA synthetase mutations must be used, which cannot be fully suppressed (compensated) by addition of the corresponding amino acid into the medium. These mutations may be produced by conventional processes for mutation induction such as by treating a prototrophic parent strain with N-methyl-N'-nitro-N-nitrosoguanidine. The screening procedure should include selection of auxotrophic mutants, which can grow only slowly on media containing high concentrations (3-10 mg/ml) of the corresponding amino acid, and cannot grow without it. This procedure permits to obtain just the mutants with impaired aminoacyl-tRNA synthetases, because the auxotrophy caused by blocks in the amino acid biosynthetic pathway can be completely compensated by addition of low concentrations (0,005-0,05 mg/ml) of the amino acid into the media.

On the basis of the mutant strains additional mutations may be obtained which increase intracellular concentration of the corresponding amino acid, by selecting (pseudo)revertants, which can grow on media containing no amino acid or containing low concentrations of the amino acid. By using this procedure different mutations in the genes involved in amino acid overproduction may be induced, and step-by-step selection leading to increased amino acid productivity may be performed. The maintainance of the defect in the aminoacyl-tRNA synthetase in revertants must have a positive effect on their productivity. Thus, effective amino acid producing strains may be prepared.

Besides, above mutations affecting aminoacyl-tRNA synthetases may be introduced into the chromosome of the amino acid producers obtained by known procedures, including selection for analogue-resistance, by transformation, transduction, conjugation or protoplast fusion to increase their productivity.

Further, above mutations affecting aminoacyl-tRNA synthetases may be introduced into the chromosome of recipient strains, which are used for the construction of amino acid producers by recombinant DNA technique.

By the method of the present invention strains were constructed which produce valine and isoleucine in higher yields than has been achieved by previously known methods using artificial mutant of Escherichia.

Having generally described this invention, a further understanding can be obtained by reference to certain specific examples.

Example 1

1. Selection of the mutants requiring a high isoleucine concentration for their growth.

Escherichia coli strain K12 W3350 was treated with N-methyl-N'-nitro-N-nitrosoguanidine and cultured with shaking in the presence of 10 mg/ml of L-isoleucine at 37°C for 24 hours. The cells were then washed, resuspended in minimal medium supplemented with glucose (0.2 %) and incubated at 37°C. When a doubling (A590) was obtained, 2000 units of penicillin per ml. were added, and after 3 hours incubation at 37°C the cells were washed and spread on minimal agar plates supplemented with 0.2 % glucose and 10 mg/ml L-isoleucine. Isoleucine auxotrophs which can grow only in the presence of high isoleucine concentration in the medium were selected using a conventional procedure. Three independent mutants which have thus been isolated, are : IleS 2, IleS 17 and IleS 32.

Table 1 shows the optical densities (A590) of the 18 hours cultures of the isoleucine-requiring mutants grown on minimal medium supplemented with different isoleucine concentrations.

## TABLE 1

| Strain | isoleucine (mg/ml) | | | | | |
|--------|------|------|------|------|------|------|
|        | 0.00 | 0.01 | 0.05 | 1.0 | 3.0 | 5.0 |
|        | Optical density (A590) | | | | | |
| IleS 2 | 0.001 | 0.18 | 0.33 | 1.10 | 1.30 | 1.60 |
| IleS 17 | 0.001 | 0.013 | 0.04 | 0.11 | 0.41 | 0.76 |
| IleS 32 | 0.001 | 0.14 | 0.29 | 1.00 | 2.00 | 2.00 |
| VL 330 | 0.001 | 0.93 | 1.70 | 1.60 | 1.40 | 1.40 |
| W 3350 | 1.50 | 1.70 | 2.00 | 2.00 | 2.00 | 2.00 |

The regular isoleucine auxotroph VL 330 (ilvA) reaches the maximum OD at 0.005 mg/ml of isoleucine in a growth medium. The mutants IleS 2, IleS 17 and IleS 32 require much higher isoleucine concentration for their growth. Furthermore, the mutant IleS 17 grew slowly even in the presence of 5 mg/ml of isoleucine in medium.

Genetic data had shown that mutations conferring isoleucine auxotrophy to the IleS strains were mapping on the Escherichia coli chromosome between threonine and leucine operons where the gene coding for isoleucyl-tRNA synthetase is known to be located. Accordingly, isoleucyl-tRNA synthetase activities in the IleS 2 and IleS 17 strains measured by known method were 43 % and 37 % of the respective activity in the parent strain Escherichia coli W 3350.

4

2. Selection of the mutants requiring high valine concentration for their growth.

Strain of Escherichia coli VL1502 (valR, pyrB::Tn5), W 3350 derivative, was treated with N-methyl-N'-nitro-N-nitrosoguanidine and cultivated with aeration in the presence of 10 mg/ml of L-valine at 370°C for 30 hours.Then after two rounds of the penicilline enrichment procedure described above valine auxotrophs were selected. About 20 % of colonies tested were valine-requiring. Nine independent ValS mutants have thus been isolated.

Table 2 shows the optical densities (A590) of 20 hours cultures of some ValS mutants grown on minimal medium supplemented with valine in different concentrations.

## TABLE 2

| Strain | valine (mg/ml) | | | | | | | | |
|---|---|---|---|---|---|---|---|---|---|
| | 0.00 | 0.05 | 0.2 | 0.5 | 1.0 | 2.0 | 5.0 | 7.0 | 10.0 |
| | Optical density (A590) | | | | | | | | |
| ValS 52 | 0.005 | 0.01 | 0.02 | 0.03 | 0.07 | 0.16 | 1.5 | 1.8 | 1.4 |
| ValS 67 | 0.01 | 0.04 | 0.07 | 0.21 | 0.50 | 1.8 | 2.2 | 1.9 | 1.5 |
| ValS 91 | 0.02 | 0.03 | 0.04 | 0.08 | 0.15 | 0.58 | 1.9 | 1.8 | 1.45 |
| C600 ilvC * | 0.01 | 2.0 | 2.2 | 2.3 | 2.2 | 2.5 | 2.3 | 2.2 | 2.2 |

* For this strain 0.1 mg/ml of isoleucine was added together with indicated valine concentrations

All the mutations conferring valine-requirement to ValS mutants were cotransduced with high frequency by phage P1 with pyrB::Tn5 marker (82 - 96 %). This result indicates that they are located in the same region where known valS mutations were mapped. Activity of valyl-tRNA synthetase was measured by known methods in several ValS mutants and found to be decreased. In ValS 52 mutant it was only 16 % of the respective activity in the parent strain.

Thus, by using the described procedure mutants impaired in aminoacyl-tRNA synthetase activities can easily be selected.

3. Measuring of guanosinetetraphosphate level in IleS 17 mutant.

During the balanced growth IleS and ValS mutants may have decreased levels of charged isoleucyl-tRNA or valyl-tRNA due to reduced activity of the corresponding aminoacyl-tRNA synthetase. This must lead to the increased formation of guanosinetetraphosphate (ppGpp). So intracellular ppGpp concentration was measured by using a known method in the IleS 17 mutant and in the parent strain W3350 grown in minimal medium supplemented with isoleucine.

TABLE 3

| Strain | Isoleucine concentration in medium (mg/ml) | ppGpp pmole/A450 |
|---|---|---|
| W3350 | 2 | 35 |
| IleS 17 | 2<br>3<br>5<br>10 | 140<br>105<br>55<br>30 |

The results presented in table 3 show that under the same growth conditions the ppGpp concentration in IleS17 mutant is four times higher than in the strain W3350. Besides, the ppGpp level was dependent on isoleucine concentration in growth medium : the higher was the isoleucine concentration the lower was the level of ppGpp in Ile S17 cells.

4. Determination of threonine deaminase activity in IleS mutants.

Isoleucine and valine production by Escherichia coli strains must depend on the level of ilv-operon expression. To determine the effect of ileS mutations on expression of ilvGMEDA operon the threonine deaminase activity was measured by known method in ileS mutants grown on medium supplemented with isoleucine in low (0.01 or 0.05 mg/ml) and high (5.2) concentrations (Table 4).

TABLE 4

| Strain | Isoleucine concentration in medium (mg/ml) | Relative threonine deaminase activity (%) |
|---|---|---|
| IleS 2 | 0.01<br>5.20 | 757<br>87 |
| IleS 17 | 0.05<br>5.20 | 658<br>366 |
| IleS 32 | 0.01<br>5.20 | 682<br>122 |
| W3350 | 0.00<br>5.20 | 100<br>26 |

The results presented in Table 4 show that the enzyme activities in the IleS strains were higher than in the parental strain W3350 under different growth conditions. It can also be seen that in the presence of 5.20 mg/ml of isoleucine in medium the highest threonine deaminase activity was in the IleS 17 mutant (14 times higher than in the W3350).

Example 2

1. Selection of revertants of IleS strains which produce isoleucine.

The strains IleS2, IleS17 and IleS32 were treated with N-methyl-N'-nitro-N-nitrosoguanidine, spread on the plates containing minimal medium supplemented with 0.2 % glucose or 0.2 % glucose and 3 mg/ml threonine and cultured for 48-72 hours. The revertants which appeared were tested for their ability to excrete isoleucine into a medium and to feed the lawn of isoleucine-requiring mutant IlvA (syntrophism test, Table 5).

TABLE 5

| Parent strain | Number of revertants tested | Number of isoleucine excretors |
|---|---|---|
| IleS 2 | 50 | 14 |
| IleS 17 | 100 | 25 |
| IleS 32 | 75 | 24 |

Thus, using the IleS mutants isoleucine producing strains can easily be obtained.

Threonine deaminase activity and its inhibition by the end product was determined in revertants excreting isoleucine. In most of them the enzyme was not inhibited by 1 mM of isoleucine. Furthermore, in the revertant Rev 7434 (derivative of IleS 2) threonine deaminase was completely insensitive to inhibition by the end product.

Genetic and nucleotide sequence analysis of Rev 7434 had shown that the mutation conferring resistance to the inhibition was in the ilvA gene coding for threonine deaminase (ilvA7434 mutation).

2. Construction of the isoleucine producing strain containing valR, ilvA7434, and ile S17 mutations.

The strain Rev 7434 as well as other Escherichia coli K12 strains contains a mutation in the ilvG gene which decreases the ilvGMEDA operon expression. To eliminate this defect spontaneus valine-resistant mutant of this strain was obtained by plating it on agar minimal medium supplemented with glucose (0.2 %) and L-valine (1 mg/ml). Thus ValR strain VL1886 was obtained which contains a mutation conferring valine-resistance located in the ilvGMEDA operon. Phage P1 grown on the strain VL1886 was used for transduction of the strain VL334 (thrC ilvA). On minimal medium supplemented with glucose (0.2 %) and threonine (0.05 mg/ml) Ile + transductants were selected. Over 90 % of them were valine-resistant and had threonine deaminase insensitive to inhibition by isoleucine. Thus the strain VL1887 (thrC ilvA7434 valR) was obtained. Then phage P1 grown on IleS 17 was used for transduction of the strain VL1887. On minimal medium supplemented with glucose Thr + - transductants were selected. About 50 % of them grew slowly, and their growth was stimulated by isoleucine. These were transductants which received ileS 17 mutation. Thus the strain VL1892 was constructed which contains simultaneously valR, ilvA7434, and ileS 17 mutations. This strain had an elevated level of isoleucine insensitive threonine deaminase which was from 6 to 10 times higher than in Rev7434. The syntrophism test showed that the strain VL1892 produces isoleucine.

3. Construction of the isoleucine producing strain containing ilv-operons in a low copy number plasmid.

The strain AB 1206 harbours a low copy number plasmid F'14, which contains a chromosome fragment with ilv-operons and a corresponding chromosome deletion. Phage P1 grown on VL1886 was used for the transduction of AB 1206. The transductants were selected on minimal medium supplemented with glucose (0.2 %) and valine (1 mg/ml). Thus the strain KX 139 was obtained in which the F'14 plasmid contains the valR and ilv7434 mutations (that was established by measuring threonine deaminase activity by a known method). The plasmid can be transferred by conjugation in different Escherichia coli strains, more specially, in IleS 17. However, in RecA + strains it might be unstable because of the frequent integration into the chromosome. Therefore the recA mutation was introduced into the chromosome of IleS 17 strain by using the known conjugation procedure from the strain NK6659 (Hfr KL16 srl::Tn10 recA). Recombinants were obtained on L-broth agar medium supplemented with tetracycline (0.01 mg/ml) and among them the UV-sensitive IleS strain was selected. Thus the strain KX140 having genotype IleS 17 recA was prepared. The plasmid F'14 (valR ilv7434) was introduced into the cells of KX140 by conjugation to obtain the strain KX 141. The syntrophism test showed that this strain produces isoleucine.

4. Production of L-isoleucine by the novel L-isoleucine-producingstrains.

The strains of Escherichia coli VL 1892 and KX141 were inoculated by loop from the slant of an agarized M9 medium into Erlenmeyer flasks each containing 50 ml of L-broth (containing 10 g/l peptone, 5 g/l yeast extract, 1 g/l glucose and 5 g/l NaCl, pH 7.2). After inoculation the flasks were placed on a rotary shaker (200 r.p.m.) and incubated for 18 hours at the temperature of 37°C. The thus prepared material was used as a seed culture.

A main culture medium, containing 30 g/l glucose, 5 g/l ammonium sulfate, 2 g/l $K_2HPO_4$, 0.4 g/l $MgSO_4.7H_2O$, 0.02 g/l $FeSO_4 7H_2O$, 0.02 g/l $MnSO_4.5H$ 0, 2 g/l yeast autolysate, 1 g/l L-threonine was prepared.

300 ml of the medium was placed in a 0.5 l jar-fermentor and sterilized at 121°C for 15 min. The medium was inoculatd with 30 ml of the seed culture obtained above and cultivated with stirring at 900 r.p.m. and introducing 1:1 volume of air per minute. The pH of the medium was maintained automatically at around 7.2 by feeding on pH-monitor signal the mixture containing in ratio 10:7:1 10 % solution of threonine, 60 % solution of glucose, and 25 % solution of ammonia.

The fermentation was performed for 46 hours. By the end of fermentation the culture medium with VL1892 contained 8 - 13 g/l of isoleucine, and the culture medium with KX 141 contained 11 - 17.8 g/l of isoleucine. The strain KX 141 has been deposited in the All-Union Collection of Industrial Microorganisms in VNIIGENETIKA (USSR) as VKPM B-4781.

### Example 3

1. Selection of the revertants of IleS 32 ValR strain which produces L-valine.

To obtain L-valine producers the strain harbouring both IleS and valR (ilvG) mutations was constructed. The valR (ilvG) mutation restores the activity of the valine-resistant AHAS II - the key enzyme in valine biosynthetic pathway, which is defective in the strains of E. coli K12. By sequential phage P1-mediated transduction mutation valR from the strain C600 valR (resistant to 1 mg/ml of valine) and mutation ileS 32 from the strain IleS 32 were introduced into the chromosome of the strain VL334 (thrC ilvA). On the plates of agar minimal medium M9, containing glucose (0.2 %) and valine (2 mg/ml) ValR transductants were selected, and on the plates of this medium containing isoleucine (2 mg/ml) Thr + IleS transductants were obtained.

Thus the strain IleS 32 ValR was constructed. Then the revertants of this strain were selected by plating it on M9 medium containing no isoleucine. The colonies which appeared were tested for their ability to feed the lawn of isoleucine and valine requiring E. coli strain on minimal medium M9 containing isoleucine (0.05 mg/ml). Thus valine excreting strains Rev835, Rev839 and Rev 874 were obtained.

By using phage P1- mediated transduction it was found that all of the revertants contained ileS mutation which can be transferred in different Escherichia coli strains.

2. Production of L-valine by the (pseudo)revertants.

The ability of the strains obtained to produce L-valine was tested by fermentation in tubes. The fermentation medium contained 50 g/l glucose, 10 g/l ammonium sulfate, 1 g/l $K_2HPO$, 0.4 g/l $MgSO_4.7H_2O$, 0.02 g/l $FeSO_4.7H_2O$, 0.02 g/l $MnSO_4.5H_2O$ and 20 g/l $CaCO_3$ (separately sterilized) and the pH was adjusted to 7.2.

Three ml batches of the fermentation medium were placed in 20 mm tubes inoculated with one loopful inoculum of the test strain cells, and cultivation was carried out for 46 hours at 37°C.

The amount of L-valine in the supernatants of the fermentation broth is shown in Table 6.

TABLE 6

| Strain tested | L-valine produced (g/l) |
|---|---|
| Rev 835 | 4.3 |
| Rev 839 | 1.2 |
| Rev 874 | 5.6 |

3. Construction of the effective L-valine producers

To obtain more effective L-valine producers and to find out the role of IleS mutations on valine production isogenic IleS + and IleS- were constructed on the basis of Rev 874. Phage P1, cultured on the cells of donor strain NK 6066 (thr::Tn9) was used for transduction of thr::Tn9 into the chromosome of the Rev 874. The transductants were selected on L-broth agar plates, containing chloramphenicol (10 μg/ml). Among them threonine-requiring strain VL 1966 fast-growing on minimal medium containing no isoleucine

8

(IleS +) was obtained. The cells of this strain were transduced by phage P1 grown on IleS 17. The transductants were selected on M9 minimal medium containing glucose (0.2 %) and isoleucine (2 mg/ml). Among Thr + recombinants two strains, VL1968 (IleS +) and VL1970 (IleS 17) were obtained. We tested the productivity of these strains as well as of the strain Rev 874 culturing them as described above. The productivity was calculated as a ratio of valine concentration in culture medium to the optical density of the cultures. The results are presented in Table 7.

TABLE 7

| Strain tested | IleS allel | Valine productivity g/l/OD560 |
|---|---|---|
| VL1968 | ileS + | 0.01 |
| VL1970 | ileS17 | 0.76 |
| Rev 874 | ileS32 | 0.52 |

It can be seen from Table 7 that ileS mutations markedly enhance the valine productivity of E. coli valine producers. Besides, mutation ileS17 which is only partially suppressed by addition of isoleucine into the medium has a more pronounced effect on the valine productivity. The more effective valine producer strain VL 1970 was constructed by combining in one bacterial genome mutation(s) in ilv operon, which have positive effect on the valine production, with mutation ileS17, which impairs isoleucyltRNA synthetase and can be suppressed only partially by addition of exogenous isoleucine into the medium.

4. Production of L-valine by the novel Escherichia coli strain VL1970.

E. coli VL1970 was cultivated at 37°C for 18 hours in Erlenmeyer flasks with agitation (300 r.p.m.) on the seed culture medium. It was minimal medium M9 with glucose (1 %). A main culture medium, containing 30 g/l glucose, 5 g/l ammonium sulfate, 2 g/l $K_2HPO_4$, 0.4 g/l $MgSO_4.7H_2O$, 0.02 g/l $FeSO_4.7H_2O$, 0.02 g/l $MnSO_4.5H_2O$, 0.6 g/l NaCl and 0.25 g/l yeast autolysate, pH 7.2 was prepared.

300 ml of the medium was placed in a 0.5 litre jar-fermentor and sterilised at 121°C for 15 minutes. The medium was inoculated with 30 ml of the seed culture obtained above, and cultivated at 37°C with stirring at 900 rotations per minute and introducing 1:1 volume of air per minute. The pH of the medium was maintained automatically around 7.2 by introducing in the fermentor on pH-monitor signal a mixture containing 50 % glucose and 25 % ammonia in a ratio of 6:1. The fermentation was performed for 46 hours. By the end of fermentation the culture medium contained from 8.2 to 10.6 g/l of valine. This was 7 times more than obtained by using E. coli strain constructed by recombinant DNA technique.

The strain VL1970 has been deposited in the All-Union Collection of Industrial Microorganisms in VNIIGENETIKA (USSR) as VKPM B-4411.

**Claims**

1. A bacterial strain having high productivity for an amino acid, comprising in its genome a mutation which affects the corresponding aminoacyl-tRNA synthetase conferring auxotrophy which can be suppressed partially by adding the respective amino acid to the culture medium, in combination with one or more mutations which remove the negative regulation in the biosynthesis of said amino acid.

2. L-Isoleucine or L-Valine producing strain according to claim 1.

3. A bacterial strain according to claim 1 or claim 2, which belongs to the genus Escherichia coli.

4. L-Isoleucine producing mutant strain Escherichia coli VKPM B-4781 deposited in VNIIGENETIKA.

5. L-Valine producing mutant strain Escherichia coli VKPM B-4411 deposited in VNIIGENETIKA.

6. A method for constructing a bacterial strain having high productivity for an amino acid according to any of the claims 1 to 5, which comprises introducing in the genome of a bacterial strain a mutation which affects the corresponding aminoacyl-tRNA synthetase conferring auxotrophy which can be partially suppressed by adding the respective amino acid to the culture medium in combination with one or more mutations which remove the negative regulation in the biosynthesis of said amino acid.

7. A method according to claim 6, wherein the mutation which removes the negative regulation in the biosynthesis of said amino acid is obtained as (pseudo)revertant of the mutant affecting the corresponding aminoacyl-tRNA synthetase.

8. A process for producing L-isoleucine by culturing an L-isoleucine producing mutant according to any of the claims 1 to 4 in a nutrient medium and isolating L-isoleucine from the culture broth.

9. A process for producing L-valine by culturing an L-valine producing mutant according to any of the claims 1 to 3 or 6 in a nutrient medium and isolating L-valine from the culture broth.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,D | JOURNAL OF MOLECULAR BIOLOGY, vol. 22, 15th - 28th December 1966, pages 325-334, Academic Press, London, GB; J.R. ROTH et al.: "Histidine regulatory mutants in Salmonella typhimurium. II. Histidine regulatory mutants having altered histidyl-tRNA synthetase" * Page 330, lines 27-34; abstract * | 1 | C 12 N    1/20<br>C 12 N   15/00<br>C 12 P   13/06<br>C 12 P   13/08   //<br>(C 12 N    1/20<br>C 12 R    1:19 ) |
| Y | IDEM | 2-6 | |
| D,Y | JOURNAL OF BACTERIOLOGY, vol. 105, no. 2, February 1971, pages 527-537, American Society for Microbiology, Baltimore, US; M. IACCARINO et al.: "Isoleucine auxotrophy as a consequence of a mutationally altered isoleucyl-transfer ribonucleic acid synthetase" * Page 532, left-hand column, lines 16-28 * | 1-6 | |
| D,Y | JOURNAL OF BACTERIOLOGY, vol. 129, no. 1, January 1977, pages 66-70, American Society for Microbiology, Baltimore, US; E.J. JOHNSON et al.: "Threonyl-transfer ribonucleic acid synthetase and the regulation of the threonine operon in Escherichia coli" * Page 68, right-hand column, lines 9-15 *                 -/- | 1-6 | TECHNICAL FIELDS SEARCHED (Int. Cl.5)<br><br>C 12 P<br>C 12 N |

The present search report has been drawn up for all claims

| Place of search | Date of completion of the search | Examiner |
|---|---|---|
| THE HAGUE | 30-01-1992 | HORNIG H.O. |

**European Patent
Office**

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | CHEMICAL ABSTRACTS, vol. 108, no. 17, 25th April 1988, page 176, abstract no. 144395d, Columbus, Ohio, US; O.F. GAVRILOVA et al.: "Genetic mapping of the ilv7434 mutation in Escherichia coli that causes resistance of threonine deaminase to feed-back inhibition by isoleucine", & GENETIKA (MOSCOW) 1988, 24(1), 13-22 * Whole abstract * | 1-6 | |
| | --- | | |
| A | CHEMICAL ABSTRACTS, vol. 101, no. 17, 22nd October 1984, page 557, abstract no. 149704u, Columbus, Ohio, US; A.O. ARMANDZHYAN et al.: "L-valine biosynthesis by Serratia marcescens AZL-R28 auxotroph mutants", & BIOL. ZH. ARM. 1984, 37(6), 476-80 * Whole abstract * | | |
| | ----- | | |
| | | | TECHNICAL FIELDS SEARCHED (Int. Cl.5) |

The present search report has been drawn up for all claims

| Place of search | Date of completion of the search | Examiner |
|---|---|---|
| THE HAGUE | 30-01-1992 | HORNIG H.O. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P0401)